(19) Europäisches Patentamt
European Patent Office
Office européen des brevets

(11) **EP 3 357 956 A1**

(12) **EUROPEAN PATENT APPLICATION**
published in accordance with Art. 153(4) EPC

(43) Date of publication:
**08.08.2018 Bulletin 2018/32**

(51) Int Cl.:
**C08J 3/14** $^{(2006.01)}$       **G01N 33/545** $^{(2006.01)}$

(21) Application number: **16851922.1**

(22) Date of filing: **30.09.2016**

(86) International application number:
**PCT/JP2016/079161**

(87) International publication number:
**WO 2017/057753 (06.04.2017 Gazette 2017/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.09.2015   JP 2015192902**

(71) Applicants:
• **Sekisui Chemical Co., Ltd.**
  **Osaka-shi, Osaka 530-8565 (JP)**
• **Sekisui Medical Co., Ltd.**
  **Tokyo 103-0027 (JP)**

(72) Inventors:
• **SUGIMOTO, Satoru**
  **Mishima-gun**
  **Osaka 618-0021 (JP)**
• **IWAMOTO, Tadashi**
  **Mishima-gun**
  **Osaka 618-0021 (JP)**
• **WAKIYA, Takeshi**
  **Mishima-gun**
  **Osaka 618-0021 (JP)**
• **KITAHARA, Shinichiro**
  **Tokyo 103-0027 (JP)**
• **IKEGAMI, Maasa**
  **Tokyo 103-0027 (JP)**

(74) Representative: **Hoffmann Eitle**
**Patent- und Rechtsanwälte PartmbB**
**Arabellastraße 30**
**81925 München (DE)**

(54) **POLYMER MICROPARTICLES PROVIDED WITH MICROPHASE SEPARATION STRUCTURE GRAINS, REAGENT FOR PARTICLE IMMUNOASSAY USING SAME, AND PARTICLE IMMUNOASSAY METHOD**

(57)    A polymer fine particle including a block copolymer having two or more segments, wherein the polymer fine particle has a microphase separated structure on a surface thereof, the number-average particle size is 50 nm to 1000 nm, and the coefficient of variation is less than 20%. The polymer fine particle permits highly sensitive assay by controlling the antigen- or antibody-immobilized state of the particle surface.

Fig. 1

EP 3 357 956 A1

**Description**

Technical Field

The present invention relates to a polymer fine particle for particle immunoassay that permits highly sensitive assay while highly suppressing nonspecific reaction, a reagent for particle immunoassay containing the polymer fine particle, and particle immunoassay using the polymer fine particle.

Background Art

In the field of clinical examination, immunological assay exploiting antigen-antibody reaction is widely used as a method for detecting a very small amount of a substance to be detected in a specimen. Among others, particle immunonephelometry using antigen- or antibody-immobilized polymer fine particles (hereinafter, also referred to as sensitized polymer fine particles) is prevalent as a practical method because this method involves convenient assay operation, requires a short time for assay, and can be applied to instruments for clinical examination used widely. The assay of an antigen or an antibody in a specimen by particle immunonephelometry is performed by detecting optical change derived from the agglutination of the sensitized polymer fine particles associated with the formation of an immunocomplex between the antigen and the antibody. This optical change of the assay sample is based on change in apparent particle size caused by the agglutination of the sensitized polymer fine particles.

The particle immunonephelometry has often employed polystyrene polymer fine particles composed mainly of polystyrene because of easy immobilization of an antigen or an antibody specifically reactive with a substance to be detected, relatively low cost, and also easily controllable polymerization reaction. The polymer fine particles composed mainly of polystyrene, however, has the advantage that the antigen or the antibody can be simply immobilized onto the particle surface through physical adsorption, whereas the problem of these polymer fine particles is that, for the adsorption of the antigen or the antibody onto the particle surface, it is difficult to control an adsorption site or the like on the particle surface so that the antigen or the antibody cannot be adsorbed onto the particle surface with some regularity.

Patent Literature 1 describes polystyrene polymer fine particles composed mainly of polystyrene as described above.

Patent Literature 2 describes carrier particles for diagnostic reagents prepared as polymer fine particles by copolymerizing styrene and styrenesulfonate as main components with a particular surfactant having a polymerizable functional group in water using a watersoluble radical polymerization initiator. The polymer particles prepared by this method, however, have the surface covered with the surfactant, which hinders the physical adsorption of an antigen or an antibody onto the particle surface. Therefore, a diagnostic reagent containing an antigen or an antibody adsorbed on the particles cannot produce the desired sensitivity.

Patent Literature 3 describes particles for diagnostic drugs consisting of a copolymer of styrene and a monomer that provides a refractive index of 1.6 or more in a homopolymer. The surface of the particles, however, has a randomly mixed state of these two components and cannot control the adsorption of an antigen or an antibody.

Related Art

Patent Literature

Patent Literature 1: International Publication No. WO2003/005031
Patent Literature 2: Japanese Patent Laid-Open No. 57-038806
Patent Literature 3: Japanese Patent Laid-Open No. 2001-296299

Summary of Invention

Technical Problem

Under these circumstances, there has been a demand for the development of polymer fine particles for antigen or antibody immobilization or methods for producing polymer fine particles capable of controlling an antigen- or an antibody-immobilized state by devising the polymer fine particles themselves.

The polystyrene polymer fine particles composed mainly of polystyrene described above are, basically, homogeneous fine particles constituted by a polymer component of single type. Even when these fine particles contain components of different types, it is difficult to form a functional microscopic region (e.g., a hydrophobic microscopic region or a hydrophilic microscopic region) on the particle surface.

An object of the present invention is to provide a polymer fine particle for particle immunoassay that permits highly sensitive assay by controlling the antigen- or antibody-immobilized state of the particle surface, a particle immunoassay reagent containing the polymer fine particle, and particle immunoassay using the polymer fine particle.

Solution to Problem

The present inventors have conducted diligent studies to attain the object and consequently completed the present invention.

The present invention relates to the following:

[1] A polymer fine particle including a block copolymer having two or more segments, wherein the polymer fine particle has a microphase separated structure on a surface thereof, the number-average particle size is 50 nm to 1000 nm, and the coefficient of variation is less than 20%.

[2] The polymer fine particle according to [1], wherein the surface having the microphase separated structure consists of a bonding phase having a bonding moiety to a substance to be detected, and a non-bonding phase for the substance to be detected.

[3] The polymer fine particle according to [2], wherein the microphase separated structure is a sea-island or lamellar microphase separated structure.

[4] The polymer fine particle according to any one of [1] to [3], wherein the block copolymer having two or more segments is a triblock copolymer having an A-B-A structure.

[5] The polymer fine particle according to [4], wherein the triblock copolymer having an A-B-A structure is a styrene-isoprene-styrene (SIS) triblock copolymer.

[6] The polymer fine particle according to any one of [1] to [5], wherein the ratio of the bonding phase to the non-bonding phase (the bonding phase/the non-bonding phase) is 10/90 to 40/60.

[7] A reagent for particle immunoassay including a polymer fine particle according to any one of [1] to [6].

[8] A particle immunoassay method including using a reagent for particle immunoassay according to [7].

Brief Description of Drawings

[Figure 1] Figure 1 is a surface photograph (SEM photograph) of a polymer fine particle having a microphase separated structure according to Example 1.

[Figure 2] Figure 2 is a surface photograph (SEM photograph) of a polymer fine particle having a microphase separated structure and a schematic diagram illustrating an enlarged view of a portion thereof.

[Figure 3] Figure 3 is a surface photograph (SEM photograph) of a polymer fine particle having a microphase separated structure according to Reference Example 1.

Description of Embodiments

Hereinafter, the embodiments of the present invention will be described in detail.

The polymer fine particle of the present invention is a polymer fine particle comprising a block copolymer having two or more segments, wherein the polymer fine particle has a microphase separated structure on the surface thereof, the number-average particle size is 50 nm to 1000 nm, and the coefficient of variation is less than 20%.

The microphase separated structure to be formed on the surface of the polymer fine particle of the present invention is formed on the basis of the difference in physical properties between the two or more segments constituting the block copolymer and, more specifically, is based on the repulsive force between these segments. The specific shape of the microphase separated structure of the polymer fine particle of the present invention can be confirmed using an electron microscope, an optical microscope, a scanning probe microscope, small-angle X-ray (neutron) scattering, light scattering, or the like known per se in the art. Such an approach may be used in combination with a compound (e.g., osmium tetroxide) selectively reactive only with a segment constituting one of the two or more segments.

The type of the block copolymer constituting the polymer fine particle of the present invention is not particularly limited as long as the block copolymer can form microphase separation. Since the formation of the microphase separated structure is based on the difference in physical properties between the segments constituting this structure, more specifically, the repulsive force between these segments, the segments constituting the block copolymer are required to have the difference in physical properties or the repulsive force sufficient for forming microscopic regions.

The block copolymer having two or more segments forms a microphase separated structure on the particle surface. Examples of the microphase separated structure include sea-island, lamellar, gyroidal, cylindrical, and bicontinuous structures. Among them, a sea-island structure is preferred because antigens or antibodies can be arranged with fixed regularity on the particle surface.

The block copolymer having two or more segments has a bonding moiety to a substance to be detected, in at least one segment.

The segment having a bonding moiety to a substance to be detected is bonded to the substance to be detected in two modes: adsorption through chemical bonding and physical adsorption. The adsorption through chemical bonding refers to bonding through the condensation reaction of a carboxyl group or a glycidyl group present in the segment having a bonding moiety to a substance to be detected with an amino group, a hydroxy group, or a thiol group present in a protein. The physical adsorption refers to adsorption through electric interaction with aromatic hydrocarbon.

In the case of using adsorption through chemical bonding, examples of the segment having a bonding moiety to a substance to be detected include a polymer containing a monomer having a carboxyl group, such as acrylic acid or methacrylic acid, as a repeat unit, and a polymer containing a monomer having a glycidyl group, such as glycidyl (meth)acrylate, as a repeat unit.

In the case of using physical adsorption, the segment having a bonding moiety to a substance to be detected is a polymer containing an aromatic hydrocarbon-containing monomer as a repeat unit and specifically refers to, for example, styrene, $C_{1-3}$ alkyl-substituted styrene such as methylstyrene, halogen-substituted styrene such as chlorostyrene, carboxy-substituted styrene such as 4-vinylbenzoic acid, sulfonic acid-substituted styrene such as styrenesulfonic acid, a compound having a condensed ring of styrene, such as vinylnaphthalene, or a polyfunctional vinylbenzene such as divinylbenzene. The position on the benzene ring at which the substituent may be added can be any of positions 2, 3, and 4. Also, the carboxy-substituted styrene or the sulfonic acid-substituted styrene may form a salt with sodium, potassium, or the like.

Specific examples of the segment lacking the bonding moiety to a substance to be detected include a highly or low polar monomer having no chemical bonding site, such as methyl methacrylate, methyl acrylate, isoprene, butadiene, ethylene, butylene, n-butyl (meth)acrylate, t-butyl (meth)acrylate, dicyclopentenyl acrylate, dicyclopentenyloxyethyl acrylate, and dicyclopentanyl acrylate. Among them, methyl methacrylate, isoprene, or butadiene is preferred.

The block copolymer having two or more segments is not particularly limited and is preferably a diblock copolymer having an A-B structure, a triblock copolymer having an A-B-A structure, a triblock copolymer having an A-B-C structure, or the like because these block copolymers are conveniently synthesized and are obtained as many commercially available products.

Examples of the diblock copolymer having an A-B structure include a styrene-isoprene diblock copolymer and a styrene-methyl methacrylate diblock copolymer.

Examples of the triblock copolymer having an A-B-A structure include triblock copolymers such as a styrene-isoprene-styrene (SIS) triblock copolymer, a styrene-butadiene-styrene (SBS) triblock copolymer, an isoprene-styrene-isoprene triblock copolymer, and a butadienestyrene-butadiene triblock copolymer.

Examples of the triblock copolymer having an A-B-C structure include triblock copolymers such as a styrene-butadiene-methyl methacrylate triblock copolymer.

These block copolymers may be synthesized by methods known in the art, or commercially available products may be used. Examples of the methods known in the art include reversible addition-fragmentation chain transfer (RAFT) polymerization, atom transfer radical polymerization (ATRP), tellurium-mediated radical polymerization (TERP; living radical polymerization method using an organotellurium compound), nitroxide-medicated radical polymerization (NMP), living radical polymerization using an iniferter or the like, living anion polymerization, living cation polymerization, living coordination polymerization, and transfer polymerization.

Examples of the commercially available products of the diblock copolymer having an A-B structure include poly(styrene-b-butadiene) (manufactured by Polymer Source Inc., P2124-SBd, average molecular weight: 74100, content of the bonding moiety to a substance to be detected: 12.2 w/w%), poly(styrene-b-methyl methacrylate) (manufactured by Polymer Source Inc., P18258P-SMMA, average molecular weight: 391000, content of the bonding moiety to a substance to be detected: 14.0 w/w%), and poly(vinylbenzyl chloride-b-methyl methacrylate) (manufactured by Polymer Source Inc., P19317B-4VBCMMA, average molecular weight: 79000, content of the bonding moiety to a substance to be detected: 30.3 w/w%).

Examples of the commercially available products of the triblock copolymer having an A-B-A structure can include: SIS triblock copolymers such as product name D1114P (manufactured by Kraton Polymers Japan Ltd., catalog No. K0169, content of the bonding moiety to a substance to be detected: 19.0 w/w%) and product name D1164P (manufactured by Kraton Polymers Japan Ltd., catalog No. K0377, content of the bonding moiety to a substance to be detected: 29.0 w/w%); and SBS triblock copolymers such as product name D1116B (manufactured by Kraton Polymers Japan Ltd., catalog No. K0037, content of the bonding moiety to a substance to be detected: 23.0 w/w%) and poly(methyl methacrylate-b-styrene-b-methyl methacrylate) (manufactured by Polymer Source Inc., P18287C-MMASMMA, average molecular weight: 82000, content of the bonding moiety to a substance to be detected: 16.5 w/w%) .

Examples of the commercially available products of the triblock copolymer having an A-B-C structure can include styrene-butadiene-methyl methacrylate triblock copolymer (manufactured by Polymer Source Inc., P8925-SBdMMA, average molecular weight: 330000, content of the bonding moiety to a substance to be detected: 14.0 w/w%).

For the block copolymer having two or more segments, preferably, the weight-average molecular weight of the triblock copolymer is 3000 to 3000000, and the content of the bonding moiety to a substance to be detected is 5 to 50 w/w%. The weight-average molecular weight is more preferably 6000 to 1000000, further preferably 9000 to 100000. The content of the bonding moiety to a substance to be detected is more preferably 10 to 40 w/w%, further preferably 15 to 35 w/w%.

The content of the bonding moiety to a substance to be detected means the weight ratio of a monomer unit having the bonding moiety to a substance to be detected to the total weight of the polymer. The content of the bonding moiety to a substance to be detected can be measured by pyrolysis gas chromatography or the like.

In the polymer fine particle of the present invention, the bonding phase for a substance to be detected and a non-bonding phase for the substance to be detected form the microphase separated structure. The area ratio of the microscopic region of the protein-bonding moiety phase to the microscopic region of the non-bonding phase for the substance to be detected, on the polymer fine particle surface is 5/95 to 50/50. The area ratio of the microscopic region of the protein-bonding moiety phase to the microscopic region of the non-bonding phase for the substance to be detected, on the polymer fine particle surface is more preferably 10/90 to 40/60, further preferably 15/85 to 35/65.

The method for controlling the area ratio is achieved by controlling the composition of the block copolymer constituting the polymer fine particle. Specifically, the area ratio of the protein-bonding phase substantially agrees with the weight ratio of the monomer unit having the bonding moiety to a substance to be detected to the total weight of the polymer.

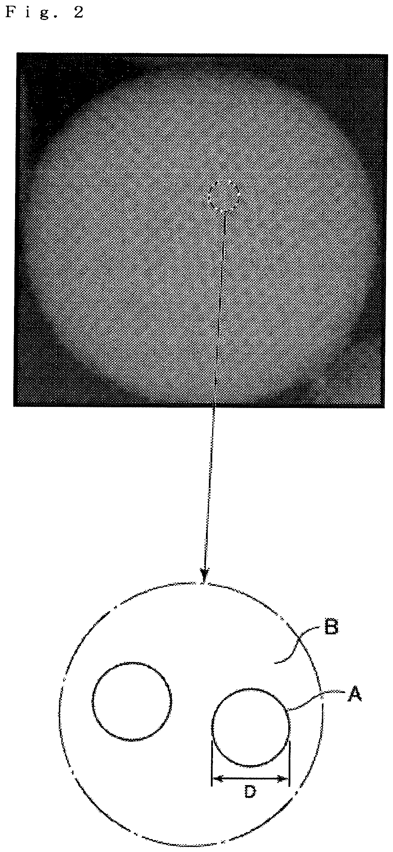

Figure 2 illustrates a surface photograph (SEM photograph) of the polymer fine particle having a microphase separated structure and a schematic diagram of an enlarged view of a portion thereof. When the microphase separated structure is a sea-island structure, it is preferred that, as illustrated in Figure 2, island phase separated structure (island microscopic region) A should be formed by the bonding phase for the substance to be detected while sea phase separated structure (sea microscopic region) B should be formed by the non-bonding phase for the substance to be detected. In the sea-island polymer fine particle illustrated in Figure 2, diameter D of the island phase separated structure A is preferably 1 to 100 nm, more preferably 3 to 50 nm, further preferably 5 to 30 nm. Within the range described above, the efficiency can be further enhanced because one antibody molecule is immobilized on one island phase separated structure.

Since the island microscopic region forms the bonding moiety to a substance to be detected, an antibody or the like against the substance to be detected can be selectively adsorbed thereon. Unlike conventional polymer fine particles, the polymer fine particle of the present invention is less likely to cause the uneven adsorption of the antibody or the like onto the particle surface. On the other hand, since the sea phase separated structure forms the non-bonding phase for the substance to be detected, the need of blocking treatment as in conventional polymer fine particles is low, as a matter of course. For such reasons, the polymer fine particle of the present invention can achieve both the suppression of nonspecific reaction and highly sensitive assay.

The polymer fine particle of the present invention having a microphase separated structure formed on the surface is obtained by, for example, a particle preparation method using polymers as starting materials or a method for synthesizing particles by polymerization using monomers as starting materials. Examples of the former method include a reprecipitation method, a solvent diffusion method, a drying-in-liquid method, and a self-assembly deposition method. Examples of the latter method include emulsion polymerization, dispersion polymerization, and miniemulsion polymerization.

The average particle size of the polymer fine particle of the present invention is 1000 nm or smaller, preferably 50 nm to 1000 nm. If the average particle size is smaller than 50 nm for use in particle immunonephelometry, sensitivity necessary for assay cannot be obtained due to too small an amount of optical change derived from the agglutination of polymer fine particles. This requires a lot of time for centrifugation in reagent preparation and increases reagent cost. If the average particle size is larger than 1000 nm, the amount of optical change caused by the agglutination of polymer fine particles exceeds a measurable range at a high concentration of the substance to be detected so that the amount of optical change in response to the amount of the substance to be detected cannot be obtained. The average particle size differs depending on an assay method or an assay instrument using the polymer fine particle of the present invention and is preferably 50 nm to 700 nm, more preferably 50 nm to 400 nm.

The coefficient of variation (CV value) of the particle size of the polymer fine particle is preferably 20% or less. If the coefficient of variation exceeds 20%, lot reproducibility may be poor in reagent preparation, resulting in reduction in assay reagent reproducibility. The coefficient of variation is more preferably 15% or less, further preferably 10% or less. The coefficient of variation of the particle size is calculated according to the following expression:

$$\text{Coefficient of variation (CV value) of the particle size} = \text{Standard deviation of the particle size} / \text{Average particle size.}$$

Reportedly, a polymer fine particle comprising a block copolymer having two or more segments forms a microscopic region by a microphase separated structure on the surface thereof. For example, Japanese Patent Laid-Open No. 2006-077076 describes a polymer fine particle comprising a styrene-isoprene (SI) diblock copolymer and having a lamellar microphase separated structure on the surface thereof. There is also a report stating that the particle size distribution of SI diblock copolymer particles prepared by this method is approximately 100 nm to 600 nm (Higuchi et al.; Macromolecular Rapid Communications, 31, 1773-1778 (2010)). In spite of the report on a polymer fine particle comprising a block copolymer and having a phase separated structure formed on the surface thereof, the particle size distribution is broad for use in immunoassay. No practical polymer fine particle has been reported so far.

In this respect, the polymer fine particle of the present invention has the predetermined particle size and particle size distribution as mentioned above. Furthermore, the bonding moiety mentioned later can be immobilized onto the particle surface with reproducibility. As a result, the polymer fine particle of the present invention is applicable to immunoassay through the use of the difference in properties between the microscopic regions.

The polymer fine particle of the present invention is obtained in a state suspended in the poor solvent, and therefore used in a state suspended in an appropriate aqueous medium such as a buffer solution for preparing a reagent for particle immunonephelometry. The concentration of the particle is not particularly limited and is usually preferably 1 to 20 w/v%. If the concentration is lower than 1 w/v%, enrichment is necessary for reagent preparation. If the concentration is higher than 20 w/v%, particles may be agglutinated.

A polymer fine particle in which a substance (hereinafter, also referred to as a "bonding moiety") specifically reactive with the substance to be detected through physical adsorption is immobilized on the surface (sensitized polymer fine particle), and a reagent for particle immunonephelometry comprising the polymer fine particle are also one aspect of the present invention. The "substance specifically reactive with the substance to be detected" is not particularly limited as long as the substance can be used in immunological agglutination reaction and agglutination inhibition reaction. Among others, a substance that can be used in antigen-antibody reaction is preferred.

Examples of the substance that can be used in antigen-antibody reaction include antigens such as proteins, nucleic acids, lipids, and hormones, and antibodies against these antigens. The antigens are not particularly limited and can be antigens suitable for the detection of antibodies in specimens, for example, various allergens, various steroid hormones, syphilis lipid antigens, antigens derived from various bacteria, and toxin antigens. Examples of the antibodies include, but are not particularly limited to, antibodies against substances whose abundance varies depending on various causes of diseases or pathological conditions, for example, pathogen- or disease-specific molecular markers. Specific examples of these antibodies include antiinfluenza antibodies, anti-AFP antibodies, anti-CRP antibodies, anti-insulin antibodies, and anti-BNP antibodies. The antibodies may be immunoglobulin molecules themselves or may be fragments, for example, $F(ab')_2$. The antibodies may be any of polyclonal antibodies and monoclonal antibodies for use and are not limited by methods for producing the same.

The method for immobilizing the substance specifically reactive with the substance to be detected onto the polymer fine particle (or sensitizing the polymer fine particle with the substance specifically reactive with the substance to be detected) is not particularly limited as long as the method is a physical adsorption method. The immobilization can be performed by a conventional method known in the art.

After the immobilization, the polymer fine particle can be subjected, if necessary, to blocking treatment with bovine serum albumin (BSA) or the like and dispersed in an appropriate buffer solution to prepare a sensitized polymer fine particle dispersion. This sensitized polymer fine particle dispersion can be combined with buffer solutions and standard substances, etc. for use in assay and used as a reagent (kit) for particle immunonephelometry.

The polymer fine particle of the present invention can produce favorable assay results even without being subjected to blocking treatment. Some substances to be detected may be easily adsorbed onto the particle surface. Therefore, the blocking treatment may be performed, if necessary. The blocking agent can be selected from substances known in the art, for example, bovine serum albumin, skimmed milk, fish gelatin, and surfactants. Among them, bovine serum albumin is preferably used.

In the case of using these substances, it is desirable that blocking treatment time, warming conditions, concentration, etc. should be appropriately selected according to the substance to be blocked, the particle surface state, etc.

The amount in which the substance (bonding moiety) specifically reactive with the substance to be detected is immobilized on the polymer fine particle differs depending on the type of the substance specifically reactive with the test substance used and is not particularly limited.

The immobilized amount of the substance specifically reactive with the substance to be detected can be determined, for example, by mixing the particle with the substance specifically reactive with the substance to be detected to immobilize the substance specifically reactive with the substance to be detected onto the particle, then performing centrifugation, and measuring the residual amount of the substance specifically reactive with the substance to be detected in the supernatant without being immobilized on the particle.

For use of an assay reagent comprising the polymer fine particle for the immobilization of an antigen, an antibody, or the like, the assay reagent may contain various sensitizers for improvement in assay sensitivity or acceleration of antigen-antibody reaction. Examples of the sensitizers include alkylated polysaccharides such as methylcellulose and ethylcellulose, pullulan, and polyvinylpyrrolidone.

Use of the polymer fine particle of the present invention highly suppresses nonspecific reaction. In order to further suppress nonspecific reaction which is caused by other substances present in a specimen or enhance the preservation stability of the reagent, the assay reagent may contain a component known in the fields of immunological assay methods and reagents using polymer fine particles, for example, a protein such as albumin (bovine serum albumin or egg albumin), casein, or gelatin, a decomposition product thereof, a peptide, an amino acid, or a surfactant.

The substance to be detected may be diluted with an appropriate diluent. Any buffer solution of pH 5.0 to 9.0 can be used as the diluent. Examples thereof include phosphate buffer solutions, glycine buffer solutions, Tris buffer solutions, borate buffer solutions, and citrate buffer solutions.

Use of the assay reagent comprising the polymer fine particle for the immobilization of an antigen, an antibody, or the like of the present invention can measure the reacted amount of the substance to be detected in a specimen by optically measuring the degree of agglutination of the polymer fine particle resulting from the reaction of the substance to be detected in the specimen with the substance specifically reactive with the substance to be detected, immobilized on the polymer fine particle. The optical measurement can employ any general instrument for clinical examination typified by, for example, an optical instrument capable of detecting scattered light intensity, transmitted light intensity, absorbance, etc., or an optical instrument provided with a plurality of these detection methods.

A conventional method known in the art is used as a method for optically measuring the degree of agglutination. Examples thereof include nephelometry of detecting the formation of agglutination as increase in turbidity, a method for detecting the formation of agglutination as change in particle size distribution or average particle size, and integrating sphere turbidity assay of measuring change in forward scattered light caused by the formation of agglutination using an integrating sphere and comparing a ratio to transmitted light intensity.

Examples of the method for measuring the amount of change in the degree of agglutination include rate assay of obtaining at least two measurement values at different points in time and determining the degree of agglutination on the basis of increment (increased rate) in the measurement values between these points in time, and endpoint assay of obtaining one measurement value at a certain point in time (usually, a point in time considered to be the endpoint of reaction) and determining the degree of agglutination on the basis of this measurement value. Among them, endpoint assay based on nephelometry is preferred in terms of the convenience and rapidness of the assay.

Although the particle immunonephelometry is described above, the polymer fine particle of the present invention can also be used in membrane immunoassay such as lateral flow assay or immunochromatography, for example, by allowing the fine particle to contain, be bound with, or be covered with a dye or a metal.

The term "particle immunoassay" used in the present specification include both particle immunonephelometry and membrane immunoassay. In the present specification, the polymer fine particle of the present invention containing, bound with, or covered with a dye or a metal as described above is also referred to as the composite particle of the present invention.

A conventional method known in the art, including a method described in Japanese Patent Laid-Open No. 2010-185023 can be appropriately selected as a method for allowing the polymer fine particle of the present invention to contain, be bound with, or be covered with a dye or a metal, and used by modification or combination. The obtained composite particle of the present invention can also be used as reagent preparation for membrane immunoassay and assay methods according to a conventional method known in the art.

Examples

Hereinafter, the present invention will be described in more detail with reference to Examples. However, the present invention is not intended to be limited by Examples given below. The number-average particle size of the obtained polymer fine particle was measured as described below.

According to a routine method, the polymer fine particle was placed on a collodion film, and a particle image was taken under a transmission electron microscope (TEM). The particle sizes (100 particles) on the image were measured, and the number-average particle size and standard deviation were determined.

[Comparative Example 1]

(a) Production of polymer particle composed mainly of polystyrene (polystyrene particle)

A glass reaction container (capacity: 2 L) equipped with a stirrer, a reflux condenser, a temperature detector, a nitrogen inlet, and a jacket was charged with 1500 g of distilled water, 280 g of styrene, 5 g of sodium styrenesulfonate, and an aqueous solution containing 0.5 g of potassium persulfate dissolved in 10 g of distilled water. After purging of the container with nitrogen gas, polymerization was performed for 24 hours with stirring at 70°C. After the completion of the polymerization, the solution was filtered through filter paper to isolate polystyrene particles. Then, the polystyrene particles were dialyzed for 48 hours against a dialysis membrane to obtain purified polystyrene particles. The obtained polymer fine particles had a particle size of 215 nm (CV: 16.1%).

[Reference Example 1]

<Production of polymer fine particle having microphase separated structure>

(a) Block copolymer

A polystyrene-polyisoprene (SI) diblock copolymer [Mn: polystyrene (17,800)-b-polyisoprene (12,000); Mw/Mn = 1.02; manufactured by Polymer Source, Inc.] was used.

(b) Production of polymer fine particle

The SI diblock copolymer was dissolved in a tetrahydrofuran (THF) solution (containing a stabilizer; manufactured by Wako Pure Chemical Industries, Ltd., EP grade) to prepare a THF solution having a concentration of 0.1 g/L. This THF solution was mixed at THF solution:Milli-Q water = 1:2. Then, the mixture was left at ordinary pressure and room temperature for the complete evaporation of the good solvent THF to obtain a polymer fine particle dispersion. The dispersion was filtered through filter paper to isolate the obtained polymer fine particles. Then, the polymer fine particles were dialyzed for 48 hours against a dialysis membrane to obtain purified polymer fine particles. The obtained polymer fine particles had a particle size of 400 nm (CV: 40.3%).

(c) Structure of polymer fine particle

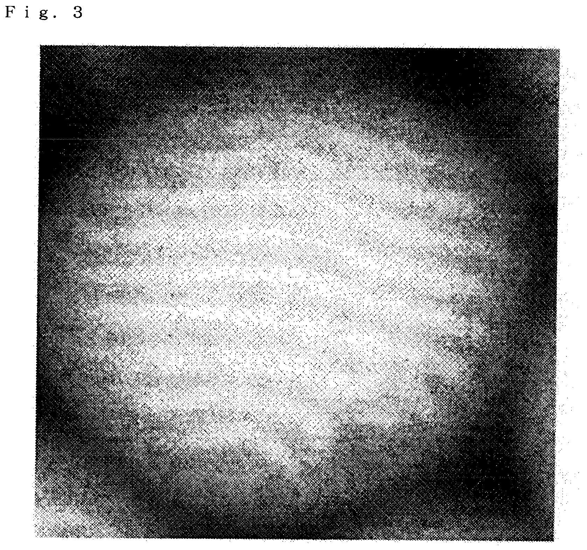

The obtained polymer fine particles comprising the SI diblock copolymer were stained with osmium tetroxide selectively reactive only with the polyisoprene block segment and then observed under FE-SEM (S-4800, manufactured by Hitachi High-Technologies Corp.) equipped with a highly sensitive YAG reflection electron detector. Figure 3 is a reflection electron image and illustrates that osmium was present in the white part. It was confirmed that a lamellar periodic microphase separated structure was formed on the surface of this polymer fine particle.

[Example 1]

<Production of polymer fine particle of present invention having microphase separated structure>

(a) Block copolymer

A polystyrene-polyisoprene-polystyrene (SIS) triblock copolymer [D1114P, Mn: polystyrene (8000)-b-polyisoprene (26000)-b-polystyrene (5000); Mw/Mn = 1.04; manufactured by Polymer Source, Inc.] was used.

(b) Production of polymer fine particle

1.0 g of the SIS triblock copolymer was dissolved in 10 mL of ethyl acetate to obtain a polymer solution. This polymer solution was added to 50 mL of water and emulsion-dispersed using an ultrasonic homogenizer. The obtained emulsion was heated and decompressed under conditions of 50°C, 6 hours, and 0.1 MPa in a reactor with a decompression apparatus for the removal of ethyl acetate to obtain a polymer fine particle dispersion. The obtained polymer fine particles were repetitively centrifuged in water to remove particulates. The obtained solution was filtered through filter paper to isolate the obtained polymer fine particles. Then, the polymer fine particles were dialyzed for 48 hours against a dialysis membrane to obtain purified polymer fine particles. The obtained polymer fine particles had a particle size of 210 nm (CV: 18.9%).

(c) Structure of polymer fine particle

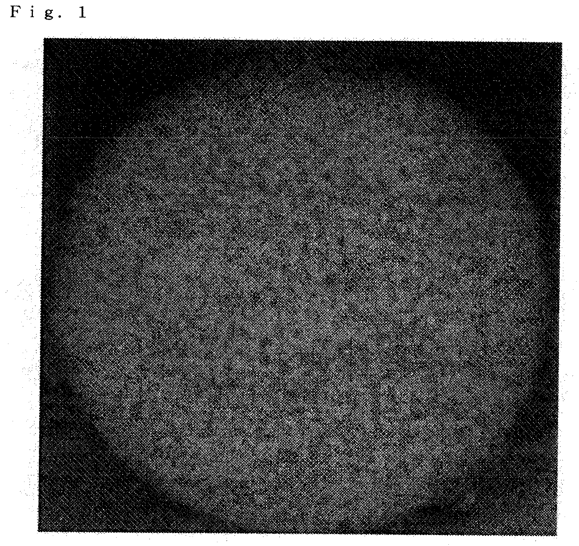

The obtained polymer fine particles comprising the SIS triblock copolymer were stained with osmium tetroxide selectively reactive only with the polyisoprene block segment and then observed under FE-SEM (S-4800, manufactured by Hitachi High-Technologies Corp.) equipped with a highly sensitive YAG reflection electron detector. Figure 1 is a reflection electron image and illustrates that osmium was present in the white part. It was confirmed that a sea-island microphase separated structure was formed on the surface of the polymer fine particle of the present invention.

[Example 2]

<Production of polymer fine particle of present invention having microphase separated structure>

(a-2) Monomer

Styrene [manufactured by Wako Pure Chemical Industries, Ltd.] was used as a monomer having a bonding moiety to a substance to be detected, and t-butyl methacrylate [manufactured by Wako Pure Chemical Industries, Ltd.] was used as a monomer having no bonding moiety to a substance to be detected.

(b-2) Production of polymer fine particle

A glass reaction container (capacity: 50 mL) equipped with a stirrer, a reflux condenser, a temperature detector, a nitrogen inlet, and a jacket was charged with 12 g of distilled water, 8 g of ethanol, 1.4 g of t-butyl methacrylate, 0.0036 g of S-dodecyl-S'-($\alpha,\alpha$'-dimethyl-$\alpha$"-acetic acid)trithiocarbonate, and 0.00012 g of 2,2'-azobis(2,4-dimethylvaleronitrile). After purging of the container with nitrogen gas, polymerization was performed for 48 hours with stirring at 80°C. Then, 1.0 g of styrene was added thereto, and polymerization was further performed for 48 hours with stirring at 80°C. After the completion of the polymerization, the particles were swollen by the addition of 10 ml of tetrahydrofuran and then stirred at room temperature for 24 hours to remove THF. Solid matter was recovered from the obtained solution in a centrifuge, repetitively washed with water, and then dialyzed for 48 hours against a dialysis membrane to obtain purified phase separated particles. The obtained phase separated particles had a particle size of 300 nm (CV: 10.3%).

(c-2) Structure of polymer fine particle

The obtained polymer fine particles comprising the block copolymer were stained with ruthenium tetroxide selectively reactive only with the poly-t-butyl methacrylate block segment and then observed under FE-SEM (S-4800, manufactured by Hitachi High-Technologies Corp.) equipped with a highly sensitive YAG reflection electron detector.
As described above, the polymer fine particles of Examples 1 and 2 had an average particle size and a coefficient of variation suitable for practical use for clinical examination as compared with conventional polymer fine particles.

[Application Example]

An antigen or an antibody appropriate for a substance to be detected is immobilized onto the polymer fine particle of the present invention to prepare an assay reagent for the substance to be detected. The controlled state of the antigen or the antibody immobilized on the particle can be confirmed by assay using an automatic analysis apparatus for clinical examination used widely.
The following reagents and material were used.

<Reagent and material>

Anti-D dimer antibody

Buffer solution for antibody-immobilized polymer fine particle preparation: 20 mmol/L Tris-HCL (pH 8.0) was used.
Buffer solution for blocking: 20 mmol/L Tris-HCL (pH 8.0) containing 2% BSA was used.
Buffer solution for specimen dilution: 30 mmol/L Tris-HCL (pH 8.5) containing 0.15% BSA was used.

<Preparation of reagent for D dimer assay>

The polymer fine particles obtained in each of Example 1 and Comparative Example 1 were purified by centrifugation and then diluted into 5% (w/v) with the buffer solution for antibody-immobilized polymer fine particle preparation to prepare a polymer fine particle dilution.

On the other hand, the anti-D dimer antibody was diluted into a concentration of 0.1 mg/mL with the buffer solution for antibody-immobilized polymer fine particle preparation to prepare an antibody dilution.

1 volume of the antibody dilution was added and mixed with 1 volume of the polymer fine particle dilution with stirring, and the mixture was further stirred. Then, 2 volumes of (1) the buffer solution for antibody-immobilized polymer fine particle preparation or (2) the buffer solution for blocking were further added thereto, and stirring was continued. Then, this mixture was recovered and adjusted to a polymer fine particle concentration of 0.5% (w/v) using the buffer solution for antibody-immobilized polymer fine particle preparation to prepare an antibody-immobilized polymer fine particle dispersion. Here, the dispersion prepared using (1) is referred to as "for test without blocking", and the dispersion prepared using (2) is referred to as "for test with blocking".

A calibration curve was prepared using these antibody-immobilized polymer fine particle dispersions and D dimer antigen standard solutions.

Apparatus: Hitachi automatic analysis apparatus model 7170

Wavelength used: 570/800 nm, assay temperature: 37°C

Substance to be examined (0 to 60 μg/mL D dimer standard solutions): 12 μL

First reagent (30 mmol/L Tris-HCL (pH 8.5) containing 0.15% BSA): 100 μL

Second reagent (antibody-immobilized polymer fine particle (0.5% (w/v)) dispersion): 100 μL

Light measurement point: 18-34

[Assay Example 1]

A calibration curve was prepared by assay according to the assay method described above using the antibody-immobilized polymer fine particle (0.5% (w/v)) dispersions (for test without blocking and for test with blocking) of Example 1 and Comparative Example 1. In the case of using the polymer fine particles of Example 1, highly sensitive assay with slight background agglutination was achieved, regardless of the presence or absence of blocking. On the other hand, in the case of using the polymer fine particles of Comparative Example 1, substantial sensitivity was unable to be obtained due to high background agglutination, as compared with Example 1. These results demonstrated that the polymer fine particle of the present invention is excellent in SN ratio even without blocking treatment and is useful for particle immunoassay, etc.

[Table 1]

|  |  | D dimer concentration (μg/mL) | | | | | |
|---|---|---|---|---|---|---|---|
| Polymer fine particle | | 0 | 3 | 7.5 | 15 | 30 | 60 |
| Example 1 | Without blocking | 10 | 24 | 46 | 85 | 200 | 400 |
| | With blocking | 6 | 20 | 43 | 79 | 186 | 360 |
| Comparative Example 1 | Without blocking | 75 | 78 | 85 | 92 | 230 | 425 |
| | With blocking | 25 | 34 | 56 | 98 | 185 | 372 |
| Numerical values represent mOD. | | | | | | | |

Industrial Applicability

The polymer fine particle for particle immunoassay of the present invention can be used in immunoassay exploiting antigen-antibody reaction, in particular, particle immunonephelometry using polymer fine particles.

Claims

1. A polymer fine particle comprising a block copolymer having two or more segments, wherein
the polymer fine particle has a microphase separated structure on a surface thereof,
the number-average particle size is 50 nm to 1000 nm, and
the coefficient of variation is less than 20%.

2. The polymer fine particle according to claim 1, wherein

the surface having the microphase separated structure consists of
a bonding phase having a bonding moiety to a substance to be detected, and
a non-bonding phase for the substance to be detected.

3. The polymer fine particle according to claim 2, wherein the microphase separated structure is a sea-island or lamellar microphase separated structure.

4. The polymer fine particle according to any one of claims 1 to 3, wherein the block copolymer having two or more segments is a triblock copolymer having an A-B-A structure.

5. The polymer fine particle according to claim 4, wherein the triblock copolymer having an A-B-A structure is a styrene-isoprene-styrene (SIS) triblock copolymer.

6. The polymer fine particle according to any one of claims 1 to 5, wherein the ratio of the bonding phase to the non-bonding phase (the bonding phase/the non-bonding phase) is 10/90 to 40/60.

7. A reagent for particle immunoassay comprising a polymer fine particle according to any one of claims 1 to 6.

8. A particle immunoassay method comprising using a reagent for particle immunoassay according to claim 7.

Ｆｉｇ．１

F i g . 2

Ｆｉｇ．３

## INTERNATIONAL SEARCH REPORT

| | International application No. |
|---|---|
| | PCT/JP2016/079161 |

| A. CLASSIFICATION OF SUBJECT MATTER |
|---|
| *C08J3/14*(2006.01)i, *G01N33/545*(2006.01)i |

According to International Patent Classification (IPC) or to both national classification and IPC

| B. FIELDS SEARCHED |
|---|
| Minimum documentation searched (classification system followed by classification symbols) |
| C08J3/14, G01N33/545 |

Documentation searched other than minimum documentation to the extent that such documents are included in the fields searched

| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| X | JP 2009-542862 A  (Invitrogen Dynal AS), 03 December 2009 (03.12.2009), claims; paragraphs [0019], [0022] to [0024], [0036], [0039] to [0062]; examples & US 2008/0139399 A1 claims; paragraphs [0022], [0026] to [0028], [0040] to [0066]; examples; figures & WO 2008/003099 A1    & EP 2038320 A1 & EP 2230264 A1    & CN 101511892 A | 1-4,6-8 |
| X | JP 8-134160 A  (Japan Synthetic Rubber Co., Ltd.), 28 May 1996 (28.05.1996), claims; paragraph [0026]; examples (Family: none) | 1-4 |

| ☒ Further documents are listed in the continuation of Box C. | ☐ See patent family annex. |
|---|---|

| * | Special categories of cited documents: |
|---|---|
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 16 December 2016 (16.12.16) | 27 December 2016 (27.12.16) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

International application No.

PCT/JP2016/079161

C (Continuation).   DOCUMENTS CONSIDERED TO BE RELEVANT

| Category* | Citation of document, with indication, where appropriate, of the relevant passages | Relevant to claim No. |
|---|---|---|
| X | JP 2013-97307 A  (Konica Minolta Business Technologies, Inc.), 20 May 2013 (20.05.2013), claims; paragraphs [0056] to [0063], [0078] to [0083], [0087], [0111], [0112]; example 7 (Family: none) | 1-3 |
| A | WO 2013/100146 A1  (Sekisui Medical Co., Ltd.), 04 July 2013 (04.07.2013), entire text & US 2014/0377881 A1 claims; examples; figures & EP 2799874 A1 | 1-8 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2003005031 A
- JP 57038806 A
- JP 2001296299 A
- JP 2006077076 A
- JP 2010185023 A

**Non-patent literature cited in the description**

- **HIGUCHI et al.** *Macromolecular Rapid Communications,* 2010, vol. 31, 1773-1778